# EUROPEAN PATENT APPLICATION

(11) **EP 2 301 950 A1**
(43) Date of publication of application: **30.03.2011**
(21) Application number: 09770125.4
(22) Date of filing: 22.06.2009
(51) Int. Cl.: C07K 1/22, B01J 20/22, B01J 20/34, C07C 211/53, G01N 30/88

(54) **SEPARATING AGENT FOR PURIFICATION OF PROTEIN, AND PROTEIN PURIFICATION METHOD**

(30) Priority: 23.06.2008 JP 2008163478
(71) Applicant: Tosoh Corporation, Shinnanyo-shi Yamaguchi 746-8501 (JP)
(72) Inventor: MASUMOTO, Seiji, Shunan-shi Yamaguchi 746-8501 (JP); KOMIYA, Katsuo, Shunan-shi Yamaguchi 746-8501 (JP); NAKAMURA, Koji, Shunan-shi Yamaguchi 746-8501 (JP)
(74) Representative: Browne, Robin Forsythe
(86) International application number: PCT/JP2009/061343
(87) International publication number: WO 2009/157415

(57) **Abstract**

A novel separating agent for protein purification which not only can adsorb proteins in a sufficient amount for protein purification from a low concentration buffer but also can desorb the adsorbed protein easily just by altering the pH of the buffer and a simple and economical method for its production and a method for protein purification using it.

One or two ligands selected from the group consisting of a ligand represented by the following formula (1): 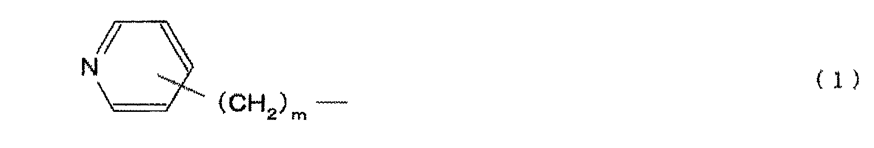 (wherein m is an integer of from 2 to 6) and a ligand represented by the following formula (2): 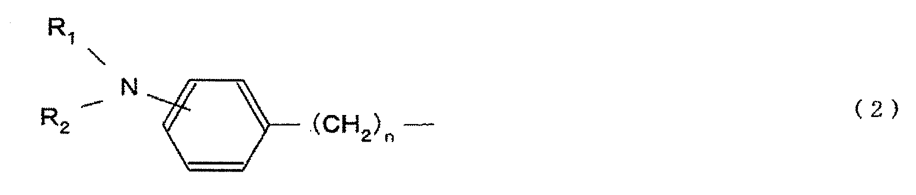 (wherein each of R₁ and R₂ is independently a hydrogen atom or a C₁₋₄ alkyl group, and n is an integer of from 1 to 6) are immobilized on a support via a urethane bond without intervention of a spacer arm.

## Description

### TECHNICAL FIELD

The present invention relates to a novel separating agent having a specific ligand for protein purification, a process for producing it and a protein purification method using it.

### BACKGROUND ART

In recent years, several techniques have been developed and/or optimized to effect separation and purification of target compounds from an aqueous mixture. Such separation and/purification techniques include, for example, ion exchange chromatography, hydrophobic interaction chromatography (HIC) (for example, Non-Patent Document 1), affinity chromatography and the like. The multiplicity of such chromatographic techniques reflects the difficulty in effecting separation and/or purification of target compounds while minimizing the complexity of the separation and/or purification procedure. Each of the techniques recited above suffer from one or more drawbacks limiting their broad use on an industrial scale.

For example, mixed mode chromatographic resins have also been employed in the art wherein such resins effect binding of a target compound under hydrophobic conditions and effect desorption of the target compound from the resin under electrostatic (ionic) or hydrophilic conditions (Non-Patent Documents 2 to 5).

One problem typical of mixed mode chromatographic resins of the prior art is that binding efficiencies of less hydrophobic target compounds to the resin is not very high unless a high salt concentration is employed in the target compound solution.

For example, in Non-Patent Document 2, protein binding to the resin on a preparative level was effected using a 1 M NaCl solution. Chromatographic techniques involving the addition of salt to an aqueous solution containing the target compound require the use of large quantities of reagents to effect recovery on an industrial scale and may necessitate substantial processing. Accordingly, chromatographic resins requiring the use of high salt concentrations are not the most efficient and cost-effective methods for recovering and/or purifying industrial quantities of such target compounds.

Patent Document 1 discloses that the use of a high ligand density on a mixed mode chromatographic resin coupled with the use of a specific ionizable ligand comprising an ionizable functionality and a spacer arm which covalently links the ligand to the solid support matrix of the resin can provide for sufficient hydrophobic character in the resin such that the target compound binds to the resin at high and low ionic strength. Patent Document 1 also discloses that target compound desorption from the resin can be achieved by hydrophilic or electrostatic (ionic) interactions by merely altering the pH of the desorbing solution such as to increase the amount of charge on the resin via the ionizable functionality.

However, because of the intervention of the spacer arm between the support (solid support matrix) and the ligand, the method described in Patent Document 1 goes through many complex processes and hence has a problem of very high production costs.

In the method described in Patent Document 1, the spacer arm refers to any group or substituent which covalently attaches the ionizable functionality to the solid support matrix. Such spacer arms include, for example, alkylene groups, aromatic groups, alkylaromatic groups, amido groups, amino groups, urea groups, carbamate groups, -R₁-Y-R₂- groups where R₁ and R₂ are alkylene groups (e.g., C₁-C₆) and Y is oxygen or sulfur and the like.

The spacer arm may be attached (linked) to the support, for example, through a neutral urethane bond by activating the solid support matrix with carbonyl diimidazole (CDI) and then reacting the activated solid support with aminocapronic acid (see Patent Document 1).

The attachment (linkage) of the ligand to the spacer arm is effected, for example, by formation of an amino bond by carbodiimide (EDC) coupling of the amine ligand to an aminocaproic resin (see Patent Document 1).

Thus, the method described in Patent Document 1 has to go through cumbersome processes for production of the chromatographic resin and has the problem that the need to adjust the pH in accordance with the ligand, the spacer arm and the solid support matrix of choice adds to the cumbersomeness of the processes.

Meanwhile, chromatographic packings for protein purification of the prior art require addition of large amounts of salt to the buffer used for protein adsorption, and it takes cost and effort to remove the salt later. Therefore, development of packings which enables protein adsorption at lower salt concentrations by a simple procedure is demanded.

### CITATION LIST

### PATENT LITERATURE

Patent Document 1: JP-A-10-506987

### NON-PATENT LITERATURE

Non-Patent Document 1: Biochimie, Vol. 60, pp.1-15 (1978)
Non-Patent Document 2: J. Chromatogr., Vol. 510, pp.149-154 (1990)
Non-Patent Document 3: J. Chromatogr., Vol. 296, pp.329-337 (1984)
Non-Patent Document 4: Chem. Rev., Vol. 89, pp. 309-319 (1989)
Non-Patent Document 5: J. Chromatogr., Vol. 317, pp. 251-261 (1984)

### SUMMARY OF INVENTION

### TECHNICAL PROBLEM

The present invention was accomplished in view of the above-mentioned background art. It is an object of the present invention to provide a novel separating agent for protein purification which can adsorb sufficient amounts of proteins for protein purification by a simple procedure using a buffer solution having a low salt concentration and enables elution of the proteins by a simple procedure only by changing the pH of the buffer, a simple and economical method for its production and a method for purifying a protein by using it.

### SOLUTION TO PROBLEM

The present inventors conducted extensive research on separating agents for protein purification and found that a separating agent having a specific ligand on a support via a urethane bond can attain the object of the present invention. The present invention was accomplished on the basis of the discovery.

Namely, the present invention provides the following separating agent for protein purification and a method for purifying a protein using it.
[1] A separating agent for protein purification which is characterized in that one or two ligands selected from the group consisting of a ligand represented by the following formula (1): 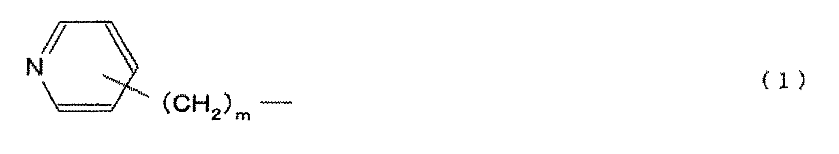 (wherein m is an integer of from 2 to 6) and a ligand represented by the following formula (2): 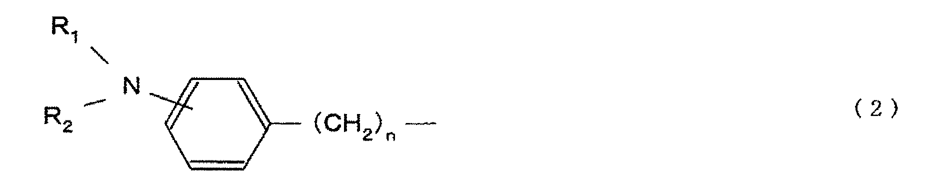 (wherein each of R₁ and R₂ is independently a hydrogen atom or a C₁₋₄ alkyl group, and n is an integer of from 1 to 6) are bonded to a support via a urethane bond.
[2] The separating agent for protein purification according to [1], wherein the ligand represented by the formula (2) is represented by the following formula (3): 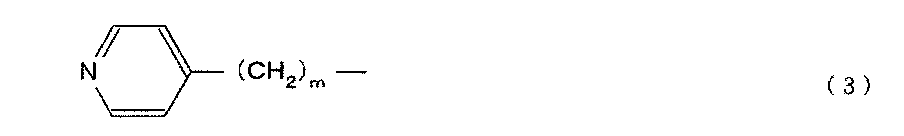 (wherein m is an integer of from 2 to 4).
[3] The separating agent for protein purification according to [1] or [2], wherein the ligand represented by the formula (2) is represented by the following formula (4): 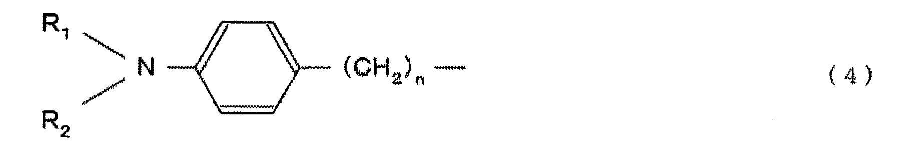 (wherein each of R₁ and R₂ is independently a hydrogen atom or a C₁₋₄ alkyl group, and n is an integer of from 1 to 4).
[4] The separating agent for protein purification according to any one of [1] to [3], wherein the amount of the ligand on the support is at least 100 µmol/ml-support.
[5] The separating agent for protein purification according to any one of [1] to [4], wherein the support is made of a crosslinked polymer.
[6] A method for producing the separating agent for protein purification as defined in any one of [1] to [5], which comprises reacting the support with an activator in an organic solvent to activate hydroxyl groups on the surface of the support, and then reacting the activated support with one or two amines selected from the group consisting of a primary amine represented by the following formula (5): 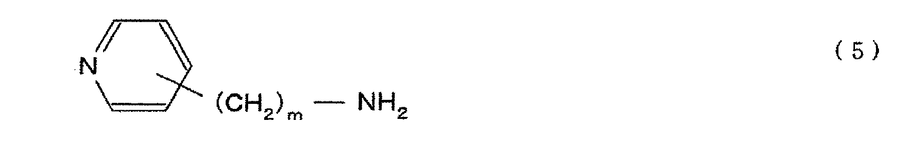 (wherein m is an integer of from 2 to 6) and a primary amine represented by the following formula (6): 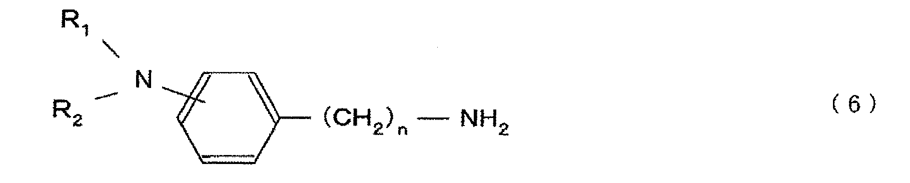 (wherein each of R₁ and R₂ is independently a hydrogen atom or a C₁₋₄ alkyl group, and n is an integer of from 1 to 6) in an organic solvent.
[7] The method for producing the separating agent for protein purification according to [6], wherein the primary amine represented by the formula (5) is represented by the following formula (7): 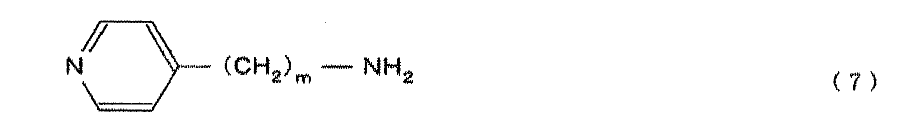 (wherein m is an integer of from 2 to 4).
[8] The method for producing the separating agent for protein purification according to [6] or [7], wherein the primary amine represented by the formula (6) is represented by the following formula (8): 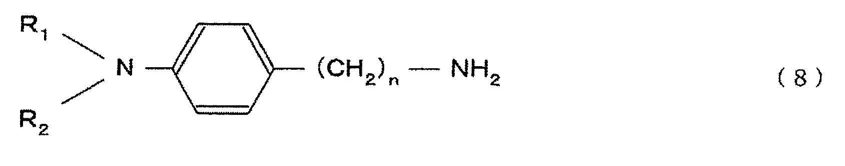 (wherein each of R₁ and R₂ is independently a hydrogen atom or a C₁₋₄ alkyl group, and n is an integer of from 1 to 4).
[9] The method for producing the separating agent for protein purification according to any one of [6] to [9], wherein the activator is carbonyldiimidazole.
[10] A method for protein purification, which comprises dissolving a protein to be purified in a first buffer, contacting the resulting protein solution with the separating agent for protein purification as defined in any one of [1] to [5] to allow the separating agent to adsorb the protein and then passing a second buffer having a different pH from the first buffer through the separating agent to elute the protein adsorbed on the separating agent.

### ADVANTAGEOUS EFFECT OF INVENTION

The separating agent for protein purification of the present invention has a specific ligand having ion exchange ability and hydrophobicity bonded on a support via a urethane bond without intervention of a spacer arm and hence can be prepared by a simple and economical production method.

Because in the separating agent for protein purification of the present invention, the ligand is immobilized on the support in a sufficient amount to adsorb proteins and have both ion exchange ability and hydrophobicity, it can easily adsorb proteins in a sufficient amount for protein purification from a low concentration buffer. Because the ligand desorbs the adsorbed protein easily just by altering the pH of the buffer, the separating agent of the present invention is especially useful as a chromatographic packing for protein purification.

### MODE OF CARRYING OUT THE INVENTION

Now, the present invention will be described in detail.

The separating agent for protein purification of the present invention is characterized in that one or two ligands selected from the group consisting of a ligand represented by the following formula (1): 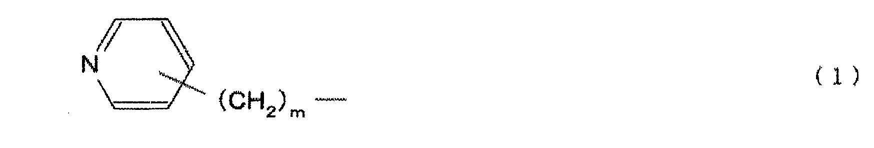 (wherein m is an integer of from 2 to 6) and a ligand represented by the following formula (2): 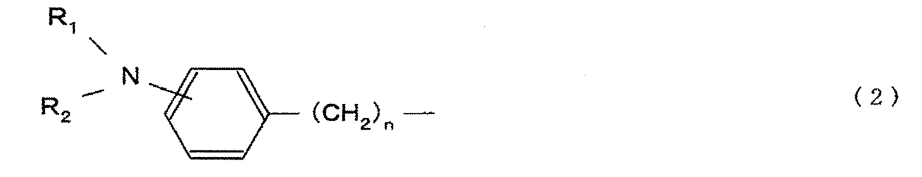 (wherein each of R₁ and R₂ is independently a hydrogen atom or a C₁₋₄ alkyl group, and n is an integer of from 1 to 6) are bonded to a support via a urethane bond without intervention of a spacer arm.

In the present invention, a ligand means a substance which specifically bind to the substance to be separated or purified.

In the present invention, the ligand represented by the above formula (1) is not particularly limited, but is preferably a ligand represented by the following formula (3): 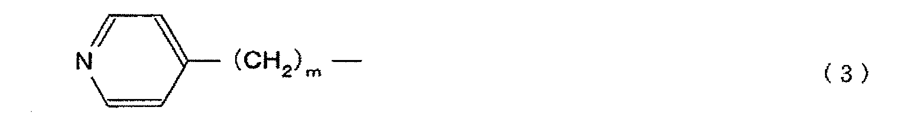 (wherein m is an integer of from 2 to 4), particularly preferably a 4-pyridylethyl group (when in the above formula (3),m is 2).

In the present invention, the ligand represented by the above formula (2) is not particularly limited, but is preferably a ligand represented by the following formula (4): 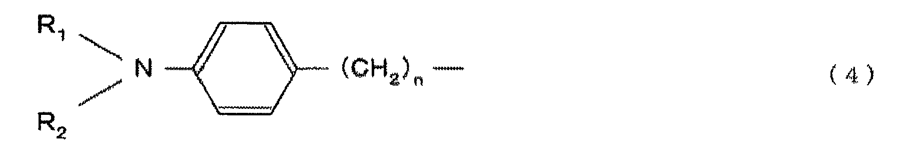 (wherein each of R₁ and R₂ is independently a hydrogen atom or a C₁₋₄ alkyl group, and n is an integer of from 1 to 4), particularly preferably a 4-aminobenzyl group (when in the above formula (4), R₁ and R₂ are hydrogen atoms, and n=1).

As the support used in the separating agent for protein purification of the present invention, known supports used as packings for column chromatography may be used without any particular restrictions. Suitable examples include inorganic porous materials such as porous glass and porous silica gel; polysaccharides such as agarose, dextran and cellulose; synthetic polymers such as polyacrylamide, polymethyl methacrylate, polyvinyl alcohol, styrene-divinylbenzene copolymers; and the like.

Because in the present invention, the above-mentioned ligand is bonded to the surface of the support via a urethane bond without intervention of a spacer arm, it is preferred that the support has hydroxyl groups on the surface. Hydroxyl groups may be introduced on the surface of the support by a known method.

Because when the separating agent for protein purification of the present invention is used on an industrial scale, the packing is washed with an alkali after purification operation, the support is preferably a alkali-resistant support made of a polysaccharide or a synthetic polymer. As the synthetic polymer, a hydrophilic vinyl polymer or a crosslinked polymer such as a crosslinked (meth)acrylate copolymer is particularly preferred.

In the present invention, although the shape of the support is dependent on how it is used and is not particularly limited, the support is in the shape of, for example, spherical particles, aspherical particles, a membrane, a monolith (continuum) or the like. For example, when used as a packing for column chromatography, it is preferably in the shape of particles, particularly preferably in the shape of spherical particles so as to be evenly filled throughout the column. It may also be a porous monolithic column integrated in a column. The separating agent may also be used in the shape of a membrane for chromatography.

When the support is used in the shape of particles, it is preferred to choose an appropriate the particle size depending on under what conditions it is used and is not particularly limited. For example, when the separating agent is used as a packing for HPLC (High Performance Liquid Chromatography), the average particle size is preferably about 5 to 15 µm, particularly preferably from 8 to 12 µm, and for the purpose of recovery of a small amount, the average particle is usually from 15 to 50 µm, preferably from 20 to 30 µm, and for use in an industrial process, the average particle size is usually 50 to 300 µm, preferably from 50 to 100 µm.

In production of the packing of the present invention, the above-mentioned ligand can be immobilized on the support via a urethane bond by a known method.

For example, the ligand can be immobilized onto the support via a urethane bond formed by reacting the support with an activator in an organic solvent to activate hydroxyl groups on the surface of the support or hydroxyl groups introduced onto the surface of the support, and then reacting the activated support with one or two amines selected from the group consisting of a primary amine represented by the following formula (5): 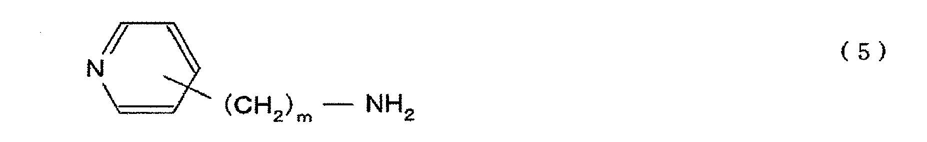 (wherein m is an integer of from 2 to 6) and a primary amine represented by the following formula (6): 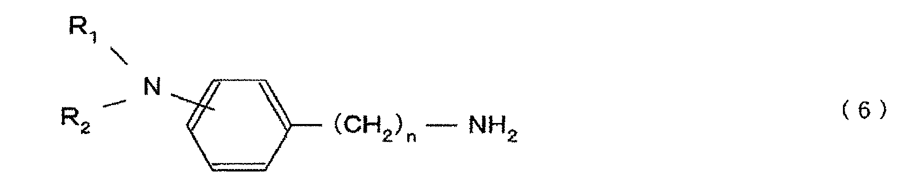 (wherein each of R₁ and R₂ is independently a hydrogen atom or a C₁₋₄ alkyl group, and n is an integer of from 1 to 6) in an organic solvent. The above-mentioned method enables simple and economical preparation of the separating agent for protein purification of the present invention.

In the immobilization method, as the activator, for example, carbonyldiimidazole (CDI) or the like is preferred, though it is not particularly limited.

The primary amine represented by the above formula (5) is preferably a primary amine represented by the following formula (7): 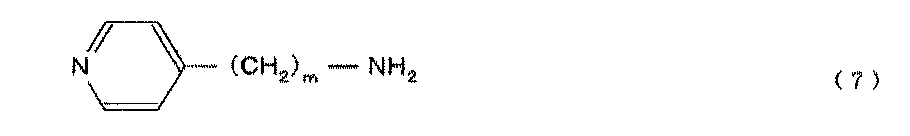 (wherein m is an integer of from 2 to 4), particularly preferably 4-(2-aminoethyl)pyridine (when in the above formula (7), m=2).

The primary amine represented by the above formula (6) is preferably a primary amine represented by the following formula (8): 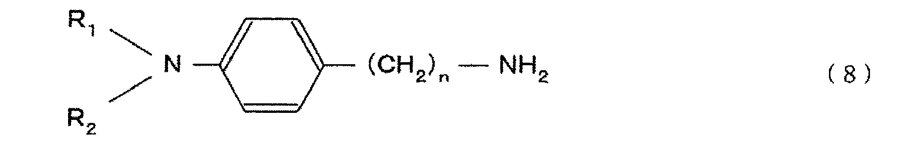 (wherein each of R₁ and R₂ is independently a hydrogen atom or a C₁₋₄ alkyl group, and n is an integer of from 1 to 4).

The organic solvent used in the series of reactions is not particularly limited, but an aprotic solvent such as 1,4-dioxane or dimethyl sulfoxide is usually used. The activation of the support with CDI is carried out usually within the range of from 15 to 30°C, preferably from 20 to 25°C, and the reaction time is usually within the range of from 1 to 3 hours. Immobilization of the above-mentioned ligand onto the support via a urethane bond by reacting the CDI-activated support with one or more amines selected from the group consisting of a primary amine represented by the above formula (5) and a primary amine represented by the above formula (6) is carried out usually within the range of from 15 to 30°C, preferably from 20 to 25°C, and the reaction time is usually within the range of from 18 to 30 hours.

The content of the ligand on the support is preferably at least 100 µmol of the primary amine per 1 ml of the support (hereinafter expressed as at least 100 µmol/ml-support), particularly preferably at least 200µmol/ml-support.

The proteins purified with the separating agent for protein purification of the present invention are not particularly limited and include, for example, immunoglobulin G (IgG), immunoglobulin A (lgA), immunoglobulin M (IgM), immunoglobulin E (lgE), albumin, chymotrypsinogen A, chymotrypsinogen B, chymotrypsinogen C, chymotrypsin Aα, chymotrypsin C, chymotrypsin inhibitors, trypsin, trypsin inhibitors, lysozyme, ribonuclease A, ribonuclease T1, ribonuclease T2, asparaginase, α-amylase, β-amylase, α-amylase inhibitor I, α-amylase inhibitor II, arginase, insulin, interferon, uricase, urokinase, estrogen receptor I, enolase, erythropoietin, leuteinizing hormone, catalase, kallikrein, kallikrein inhibitors, calcitonin, chymosin, prochymosin, papain, chymopapain A, chymopapain B, glucagon, α-glucosidase, β-glucosidase, blood coagulation factors, throid-stimulating hormone, choline esterase, concanavalin A, cytochrome b5, cytochrome b562, cytochrome c, cytochrome c2, cytochrome c550, cytochrome f, subtilisin, growth hormone, cellulose, ceruloplasmin, thyroglobulin, DNA polymerase RNA polymerase, deoxyribonuclease I, deoxyribonuclease II, deoxyribonuclease inhibitor II, transferrin, trypsinogen, thrombin, prothrombin, nuclease, neuraminidase, pancreatin, fibrinogen, parathyroid hormone, adrenocorticotropic hormone, plasminogen, plasmin, plasmin inhibitors, proteases, protease inhibitors, protein kinases, prolactin, hexokinase, pepsinogen, pepsine, hemoglobin, hemocyanin, hemopexin, peroxidase, phosphoglucomutase, phosphoglyceromutase, phospholipase A2, phospholipase C, phosphorylase a, phosphorylase b, phosphorylase kinase, polyamine oxidase, myoglobin, metallothionein, monoamine oxidase, α-lactalbumin, β-lactoglobulin, lactoferrin, lipase, lectin, renin, rhodopsin and the like. These proteins may be naturally occurring ones or recombinant ones.

The method for protein purification of the present invention comprises dissolving a protein to be purified in a first buffer having about the same salt concentration as physiological saline, contacting the resulting protein solution with the separating agent for protein purification of the present invention to allow the separating agent to adsorb the protein and then passing a second buffer having a different pH from the first buffer through the separating agent to elute the protein adsorbed on the separating agent.

In the method for protein purification of the present invention, the protein to be purified may be contacted with the separating agent for protein purification by any methods without any particular restrictions, for example, by conventional column chromatography, namely, by packing the separating agent for protein purification of the present invention into a column, loading the protein solution into the column packed with the separating agent for protein purification and then eluting the protein.

In the purification method of the present invention, the protein adsorbed by the separating agent for protein purification of the present invention may be eluted by any methods without any particular restrictions, and the adsorbed protein may be eluted, for example, by linearly changing the salt concentration and the pH of the eluent (linear gradient elution), or by changing the salt concentration and the pH of the eluent stepwise (stepwise gradient elution).

### EXAMPLES

Now, the present invention will be described in further detail with reference to Examples. However, it should be understood that the present invention is by no means restricted to such specific Examples.

TSK gel TOYOPEARL HW products (product name; manufactured by Tosoh corporation) are hydrophilic vinyl polymer-based packings for medium speed size exclusion chromatography, and TSK gel TOYOPEARL HW-65 is a hydrophilic vinyl polymer-based packing for medium speed size exclusion chromatography of proteins with an exclusion limit of about 5,000,000.

### (Determination of the Amount of the Immobilized Ligand)

TSK gel ODS-80Ts (inner diameter 0.46 cm, length 15 cm) manufactured by Tosoh Corporation was connected to a UV-8010 detector manufactured by Tosoh Corporation and a CCPM-II pump manufactured by Tosoh Corporation. An eluent was prepared by adding 0.5 (v/v)%.trifluoroacetic acid to a 40 (v/v)% acetonitrile aqueous solution. The eluent was passed through the column at a flow rate of 0.5 mL/min. Before and after immobilization, 50 µL of the primary amine suspension used for immobilization was diluted with 2 mL of the eluent, and 10 µL of the supernatant was injected. From the proportion of the peaks detected with by UV-801 0 at a wavelength of 254 nm at about 3.0 minutes, the (µmol/ml-support) amount of the primary amine used for the immobilization was determined and defined as the amount of the immobilized ligand.

### (Determination of the IgG Adsorption)

0. 5 mL of the separating agents for protein purification obtained in Examples were each packed into an open column (inner diameter 0.8 cm, length 10 cm) and equilibrated by passing 3 mL of buffer B (50 mM sodium citrate, pH 3.0) and 5 mL of buffer A (50 mM sodium phosphate containing 0.15 M sodium chloride, pH 7.2) three times. 5 mL of a 10 mg/mL human γ-globulin solution in buffer A prepared from human γ-globulin containing 150 mg/mL IgG manufactured by the Chemo-Sero-Therapeutic Research Institute was added to the column, and the column was sealed and gently shaken at room temperature for 1 hour to allow the packing to adsorb IgG. The column was washed with 5 mL of buffer A, and then, IgG was desorbed and recovered by passing 5 mL of buffer B. From the absorbance at a wavelength 280 nm measured with a UV-Visible spectrophotometer U-201 0 manufactured by Hitachi Ltd., the amount (µmol/mL-separating agent)of the IgG adsorbed on 1 mL of a separating agent for protein purification was determined and defined as the IgG adsorption.

### EXAMPLE 1

TSK gel TOYOPEARL HW-65 was sieved and immersed in 1,4-dioxane, and 10 g of the gel was weighed out in a 100 mL separable flask. Then, CDI was added in an amount of 0.70 g/g-dried TOYOPEARL, and 20 mL of 1,4-dioxane was added. The gel was stirred with a stirrer at a rotation at a number of revolutions of 200 rpm at room temperature for 1.5 hours for activation. The resulting CDI-activated TOYOPEARL was washed with 100 mL of 1,4-dioxane and transferred to a 100 mL separable flask. After addition of 20 mL of 1,4-dioxane, 4-aminobenzylamine as a primary amine was added in an amount of 0.61 g per 1 g of dry TOYOPEARL (hereinafter expressed as 0.61 g/g-dry TOYOPEARL), and immobilization was carried out at room temperature for 24 hours with stirring at a number of revolutions of 200 rpm to obtain a separating agent of the present invention.

The separating agent for protein purification having 4-aminobenzylamine immobilized thereon was washed with 30 mL of 75 mass% aqueous 1,4-dioxane, 20 mL of 33 mass% aqueous 1,4-dioxane, 100 mL of pure water, 20 mL of 0.1 N aqueous HCl and 100 mL of pure water in this order. Then, the IgG adsorption was determined by the above-mentioned method and was 50 mg/mL-packing. The amount of the immobilized ligand was determined and was 404 µmol/ml-support.

The separating agent for protein purification thus obtained was packed in stainless steel columns (inner diameter 0.75 cm, length 7.5 cm) and equilibrated with 50 mM sodium phosphate buffer containing 0.15 M sodium chloride (pH 7.2), and 200 µL of 2.5 mg/mL IgG, trypsin inhibitor, human serum albumin, bovine serum albumin and ribonuclease A and 2.0 mg/mL α-chymotrypsinogen A and lysozyme were injected, separately. All the injected proteins were adsorbed onto the separating agent for protein purification. The proteins adsorbed on the separating agent for protein purification were desorbed and recovered when the buffer was changed to 50 mM sodium citrate buffer (pH 3.0) stepwise.

### EXAMPLE 2

A separating agent for protein purification of the present invention was prepared in the same manner as in Example 1 except that 0.31 g/g-dry TOYOPEARL of 4-aminobenzylamine was used for immobilization.

The separating agent for protein purification thus obtained was washed in the same manner as in Example 1, and the IgG adsorption was determined by the above-mentioned method and was 29 mg/mL-separating agent. The amount of the immobilized ligand was determined and was 118 µmol/mL-support.

The separating agent for protein purification thus obtained was packed in stainless steel columns (inner diameter 0.75 cm, length 7.5 cm), and proteins were injected into the columns in the same manner as in Example 1. IgG, trypsin inhibitor, α-chymotrypsinogen A and lysozyme were adsorbed by the packing, whereas the other proteins passed through. The proteins adsorbed on the separating agent for protein purification were desorbed and recovered when the buffer was changed to 50 mM sodium citrate buffer (pH 3.0) stepwise.

### EXAMPLE 3

A separating agent for protein purification of the present invention was prepared in the same manner as in Example 1 except that 0.76 g/g-dry TOYOPEARL of 4-(2-aminoethyl)pyridine was used for immobilization.

The separating agent for protein purification thus obtained was washed in the same manner as in Example 1, and the IgG adsorption was determined by the above-mentioned method and was 29 mg/mL-packing.

The separating agent for protein purification thus obtained was packed in stainless steel columns (inner diameter 0.75 cm, length 7.5 cm), and proteins were injected into the columns in the same manner as in Example 1. IgG, trypsin inhibitor, α-chymotrypsinogen A and lysozyme were adsorbed by the packing, whereas the other proteins passed through. The proteins adsorbed on the separating agent for protein purification were desorbed and recovered when the buffer was changed to 50 mM sodium citrate buffer (pH 3.0) stepwise.

### INDUSTRIAL APPLICABILITY

The separating agent for protein purification of the present invention has a specific ligand having ion exchange ability and hydrophobicity bonded on a support via a urethane bond and hence can be prepared by simple and economical production method. Because the separating agent has both ion exchange ability and hydrophobicity, it not only can easily adsorb proteins in a sufficient amount for protein purification from a low concentration buffer but also can desorb the adsorbed protein easily just by altering the pH of the buffer, and hence is especially useful as a separating agent for protein purification.

The entire disclosure of Japanese Patent Application No. 2008-163478 filed on June 23, 2008 including specification, claims, drawings and summary is incorporated herein by reference in its entirety.

## Claims

1. A separating agent for protein purification which is **characterized in that** one or two ligands selected from the groups consisting of a ligand represented by the following formula (1): 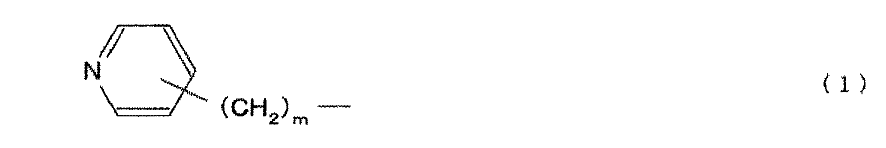 (wherein m is an integer of from 2 to 6) and a ligand represented by the following formula (2): 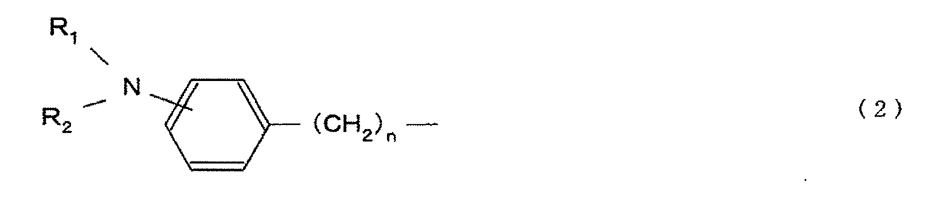 (wherein each of R₁ and R₂ is independently a hydrogen atom or a C₁₋₄ alkyl group, and n is an integer of from 1 to 6) are bonded to a support via a urethane bond.

2. The separating agent for protein purification according to Claim 1, wherein the ligand represented by the formula (2) is represented by the following formula (3): 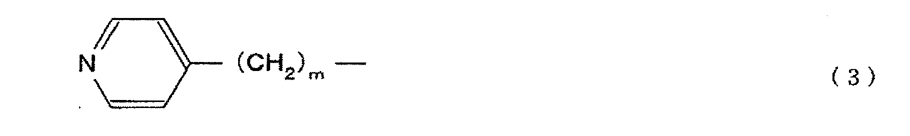 (wherein m is an integer of from 2 to 4).

3. The separating agent for protein purification according to Claim 1 or 2, wherein the ligand represented by the formula (2) is represented by the following formula (4): 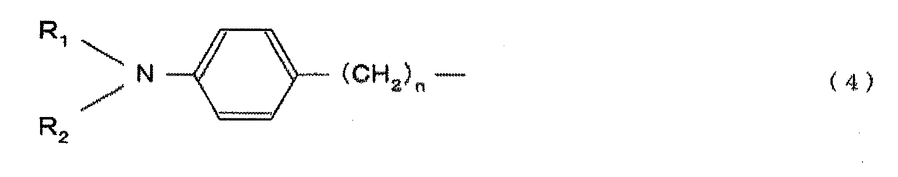 (wherein each of R₁ and R₂ is independently a hydrogen atom or a C₁₋₄ alkyl group, and n is an integer of from 1 to 4).

4. The separating agent for protein purification according to any one of Claims 1 to 3, wherein the amount of the ligand on the support is at least 100 µmol/ml-support.

5. The separating agent for protein purification according to any one of 1 to 4, wherein the support is made of a crosslinked polymer.

6. A method for producing the separating agent for protein purification as defined in any one of Claims 1 to 5, which comprises reacting the support with an activator in an organic solvent to activate hydroxyl groups on the surface of the support, and then reacting the activated support with one or two amines selected from the group consisting of a primary amine represented by the following formula (5): 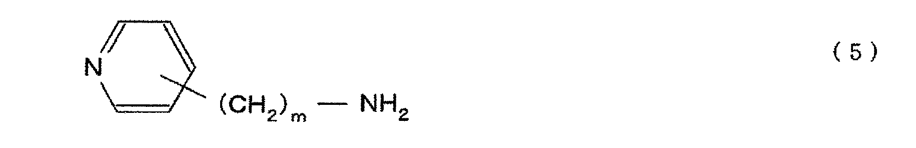 (wherein m is an integer of from 2 to 6) and a primary amine represented by the following formula (6): 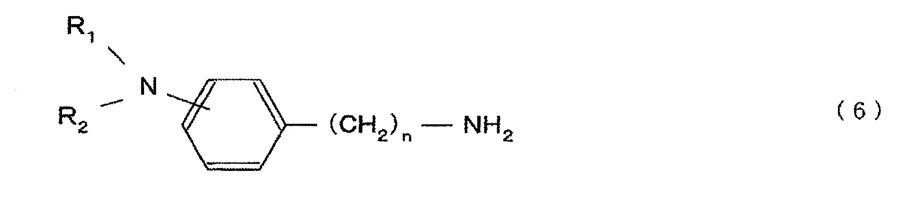 (wherein each of R₁ and R₂ is independently a hydrogen atom or a C₁₋₄ alkyl group, and n is an integer of from 1 to 6) in an organic solvent.

7. The method for producing the separating agent for protein purification according to Claim 6, wherein the primary amine represented by the formula (5) is represented by the following formula (7): 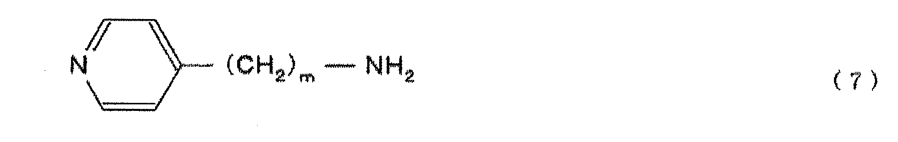 (wherein m is an integer of from 2 to 4).

8. The method for producing the separating agent for protein purification according to Claim 6 or 7, wherein the primary amine represented by the formula (6) is represented by the following formula (8): 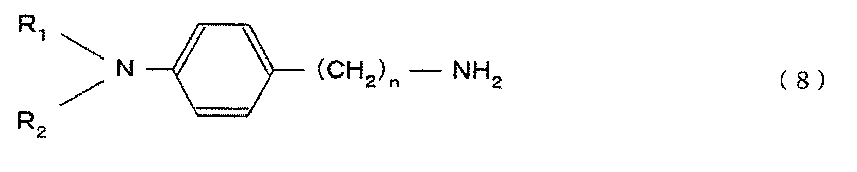 (wherein each of R₁ and R₂ is independently a hydrogen atom or a C₁₋₄ alkyl group, and n is an integer of from 1 to 4).

9. The method for producing the separating agent for protein purification according to any one of Claim 6 to 9, wherein the activator is carbonyldiimidazole.

10. A method for protein purification, which comprises dissolving a protein to be purified in a first buffer, contacting the resulting protein solution with the separating agent for protein purification as defined in any one of Claims 1 to 5 to allow the separating agent to adsorb the protein and then passing a second buffer having a different pH from the first buffer through the separating agent to elute the protein adsorbed on the separating agent.
